(19) Europäisches Patentamt

European Patent Office

Office européen des brevets

(11) EP 0 870 493 A1

## (12) EUROPEAN PATENT APPLICATION

(43) Date of publication:
14.10.1998 Bulletin 1998/42

(51) Int. Cl.$^6$: **A61K 7/16**

(21) Application number: 97302486.2

(22) Date of filing: 11.04.1997

(84) Designated Contracting States:
AT BE CH DE DK ES FI FR GB GR IE IT LI LU MC NL PT SE
Designated Extension States:
AL LT LV RO SI

(71) Applicant: CARTER-WALLACE, INC.
New York, N.Y. 10105 (US)

(72) Inventor: Fry, David R.
Hythe, Kent, CT 21 5SY (GB)

(74) Representative:
W.P. Thompson & Co.
Coopers Building,
Church Street
Liverpool L1 3AB (GB)

(54) **Oral gel composition comprising hydrated silica**

(57) There is disclosed an oral gel composition comprising in an orally acceptable vehicle the balanced admixture of at least two hydrated silicas of particular particle size, an anionic surfactant and a fluoridating agent.

EP 0 870 493 A1

Printed by Xerox (UK) Business Services
2.16.3/3.4

## Description

The present invention relates to dentifrice compositions. More particularly, the present invention relates to dentifrice compositions which are gels which exhibit superior cleaning and polishing properties. Specifically, the present invention is directed to a unique formulation containing a combination of agents for whitening and polishing teeth which maximize the whitening and polishing efficiency of the agents and which after use leave a mouth-feel similar to the feeling in the mouth usually experienced after cleaning by a dentist or dental hygienist.

More particularly, the present invention relates to a gel composition for cleaning and whitening the teeth based on the unique balanced combination of hydrated silica and an anionic surfactant such as sodium dodecylbenzenesulfonate. Humectants, thickening agents, fluoridating agents, flavors, coloring agent, sweeteners and other conventional adjuvants, such as desensitizing agents, and anticalculus agents, may also be included in the composition.

Abrasives are the solid materials added to dentifrice compositions whose functions are the removal of debris and stains from the teeth as well as the polishing of the tooth surface. An ideal abrasive material should maximize debris removal and effectively polish the teeth without causing undue abrasion to the hard tooth tissue.

Many different dentifrice compositions are known for cleaning and whitening the teeth. Of these known dentifrice compositions, many include high contents of water-insoluble abrasives which aid in removing plaque and retarding stain build-up on the teeth. However, since the ultimate goal of any oral hygiene regimen is preservation of the teeth, it is widely accepted that dentifrice compositions should include the least abrasive material necessary to remove plaque and stain.

Many materials have found utility as dental abrasives over the years, including:

Among the non-calcium cleaning and polishing agents, aluminum hydroxide, also known as aluminum hydrate and hydrated alumina, has found wide acceptance as a dental abrasive beginning from about 1926 when it was claimed in toothpaste compositions, i.e. Swedish Patent No. 69,077, followed by United States Patents Nos. 2,010,910; 2,222,969 and 2,550,207.

Aluminum hydroxide has found particular use in dentifrice formulations which contain ingredients, such as fluorides, that may be incompatible or difficulty compatible with the widely used calcium phosphates, calcium carbonate and sodium bicarbonate abrasives. The aluminum hydroxide abrasives generally have a particle size of from about 0.025 microns to about 50.0 microns.

Silica abrasives are another non-calcium abrasive which have found wide use in dentifrice compositions. The silica abrasives are polishing materials which have an average particle size ranging from about 0.1 to about 30 microns, generally between about 5 and 15 microns. The material can be precipitated silica or silica gels such as the silica xerogels described in U.S. Patent 3,862,307 and U.S. Patent 3,538,230. Widely used precipitated silica materials are those marketed by J.M. Huber Corporation under the tradename "Zeodent" or materials from the Crosfield Corporation under the tradename "Sorbosil." Silica, like alumina, is more compatible with the components of dentifrice compositions such as fluorides than are the widely used calcium phosphate, calcium carbonate and sodium bicarbonate abrasives.

According to the present invention, it has surprisingly been discovered that the combination of two hydrated silicas having distinct and different particle size ranges, one having an average particle size of from about 5 to about 15 microns, preferably from 8 to 13 microns and the other hydrated silica having an average particle size of from about 20 to about 40 microns, preferably from 20 to 30 microns, with an anionic surfactant as cleaning and polishing agents in dentifrice gel compositions maximizes the whitening and polishing efficiency and the grittiness image of the compositions, providing a mouth feel similar to that achieved after cleaning by a dentist or dental hygienist.

Moreover, the present invention is based upon the discovery that the unique combination of the whitening and polishing agent hydrated silica with the anionic surfactant (e.g. sodium dodecylbenzenesulfonate which is also an antibacterial detergent), which reduces surface tension and increases cleaning effect, yields a synergistic balanced combination which maximizes the whitening and polishing efficiency of the abrasive material.

The compositions of the invention generally include an orally acceptable vehicle (or suitable dentifrice carrier).

The dentifrices of the present invention additionally contain a fluoridating agent to aid in preventing dental caries. Many fluoridating agents suitable for use in dentifrice compositions are known. Among these are sodium, potassium, lithium or ammonium fluorides, organic amine fluorides, monofluorophosphate salts such as sodium, potassium, lithium and ammonium monofluorophosphate, sodium fluorosilicate, sodium fluorozirconate, and other materials well known to those skilled in the art. The fluoridating agents are present in an effective but non-toxic amount, e.g. in amounts of up to about 2.0%, and preferably in an amount to provide a fluoride level equivalent to range of 1000 to 1500 ppm theoretical plus a 10% excess of fluoride ion.

Suitable surfactants which impart significant antibacterial action to the composition are the anionic surfactants, preferably sodium dodecylbenzenesulfonate as well as the sulfates of long chain ($C_8$-$C_{18}$) alcohols, e.g. sodium lauryl sulfate or sodium tridecyl sulfate, the sulfates or sulfonates of monoglycerides, e.g. sodium lauroyl glyceryl or sodium coconut monoglyceride sulfonate; the sulfonates of succinic esters, e.g. sodium dioctyl sulfosuccinate; the alkyl sulfoacetates such as sodium lauryl sulfoacetate or sodium coconut sulfoacetate; the salts of sulfoacetic acid amidified with amino ethyl long chain fatty acid esters such as sodium sulfocolaurate; the amides formed from higher fatty acids with short chain amino acids such as sodium lauroyl sarcosinate or sodium methyl lauroyl tauride and soaps such as the sodium, potassium or triethanolamine salts of fatty acids.

The dentifrice composition of the present invention in addition to the combined abrasives, fluoride and antibacterial detergent may include other ingredients customarily found in dentifrice compositions.

The formulation of the invention may also include humectants. Examples of humectants include glycerol, propylene glycol, sorbitol, polyethylene glycols (generally of formula $H(OC_2H_4)_nOH$), and other materials known to those skilled in the art. The humectant may be present in the toothpaste in an amount of up to about 35%, preferably in an amount of about 8 to 25%, by weight of the dentifrice formulation. Suitable thickeners include cellulose gum, sodium carboxymethylcellulose, xanthan gum, methyl cellulose, hydroxethyl cellulose, carrageen, gum karaya, gum tragacanth, gum arabic, colloidal complex magnesium aluminum silicates (e.g. "Veegum", manufactured by R.T. Vanderbilt Co.) and sodium alginate. The thickener is incorporated in the formulation in an amount of up to about 3%, preferably within the range of about 0.3 to 1.5%, by weight of the dentifrice formulation.

In the dentifrice compositions of the present invention, the liquid vehicle may comprise water and a humectant typically in an amount ranging from about 10% to about 90% by weight of the preparation. Glycerine, propylene glycol, sorbitol, polypropylene glycol and/or polyethylene glycol (e.g. 400-600) exemplify suitable humectants/carriers. Also advantageous are liquid mixtures of water, glycerine and sorbitol.

Suitable flavoring and coloring agents include the flavoring oils, for example, peppermint, spearmint, menthol, wintergreen, clove, cinnamon, lemon, orange, methylsalicylate, licorice, or eucalyptus. The flavoring agent may be present in the dentifrice in an amount up to about 5.0% preferably in an amount within the range of about 0.3 to 2.0% by weight of the toothpaste. Useful coloring agents include FD&C Blue #1, Yellow #10 and titanium dioxide. Suitable sweeteners include sodium saccharin, sodium or calcium cyclamate, aspartame, and other sweeteners known to those skilled in the art. The sweetener may present in the toothpaste in an amount of up to about 5.0%, preferably about 0.3 to 2.0%, by weight of the toothpaste. It should be noted that humectants, e.g. sorbitol, may sweeten the formulation to some degree. However, the amount of humectant present in the composition is not included in the range of sweetener set forth above.

The preferred dentifrice formulations of the present invention comprise from about 20% to about 50% by weight of a balanced combination of hydrated silica abrasives having particle size of from 8 to 10 microns, and preferably up to about 2% of an anionic surfactant, preferably sodium dodecylbenzenesulfonate.

A number of desensitizing agents are available for the treatment of hypersensitive teeth. They included dibasic sodium citrate, potassium nitrate, strontium chloride and formaldehyde. The preferred material is potassium nitrate. The desensitizing agent may be present from 2.5% to 10.0%, with the preferable level of 5.0%.

Soluble pyrophosphates have been shown through several large scale studies to demonstrate their usefulness as inhibitors of calculus formulation. The ingredients employed as an anticalculus agent is specifically a combination of tetrapotassium pyrophosphate and tetrasodium pyrophosphate at a range of 3.0% to 7.0% total. The preferable total level is 5.9% by weight.

The formulations of the present invention are prepared by well known methods which generally comprise first mixing a gelling agent with a humectant. A water solution containing sweetener, surfactant and fluoride is added to the gelling agent/humectant mixture, then the abrasives are stirred into the mixture. Finally, the flavor oil is added and the excess air is removed.

The following example illustrate particularly preferred embodiments of the dentifrice formulations of the present invention. In all examples, all parts and percentages are given by weight of the total composition.

**EXAMPLE I**

| Ingredient | % (w/w) |
|---|---|
| Sorbitol (70% solution) | 45.4600 |
| Hydrated Silica (total) | *24.5000 |
| Aqua | 10.5494 or q.s. |
| Glycerin | 13.0000 |
| Sodium Dodecylbenzenesulfonate | 1.7600 |
| PEG-12 | 1.5000 |
| Spearment Flavor | 1.1000 |
| Sodium Monofluorophosphate | 0.7600 |
| Cellulose Gum | 0.5000 |
| Trisodium Phosphate | 0.3500 |
| Sodium Phosphate | 0.3000 |
| Sodium Saccharin | 0.2020 |
| Menthol | 0.0180 |
| FD&C Blue No. 1 Dye | 0.0006 |
| | 100.0000 |

* A combination of 15% Zeodent 113 and 9.5% Zeodent 113G

Based on the results of a Pellicle Cleaning (whitening) Study conducted at a leading U.S. Dental University (Study No 96-113), a formulation very similar to that described in the Example had a higher Pellicle Cleaning (Whitening) Ratio than did comparable, competitive whitening products currently marketed in Germany. It should be noted that the abrasivity (RDA) is comparable to other typical daily use dentifrice products which is also below the industry safety standard.

Example 2, which follows, is a particularly preferred desensitizing formulation containing potassium nitrate as the preferred desensitizing agent.

### EXAMPLE II

| Ingredient | % (w/w) |
|---|---|
| Sorbitol | 43.0000 |
| Aqua | 21.9100 or q.s. |
| Hydrated Silica | *19.0000 |
| Glycerin | 5.0000 |
| Potassium Nitrate | 5.0000 |
| Sodium Dodecylbenzenesulfonate | 1.4000 |
| PEG-12 | 1.2500 |
| Aroma 603608 | 1.0000 |
| Sodium Monofluorophosphate | 0.7600 |
| Cellulose Gum | 0.3500 |
| Sodium Fluoride | 0.1100 |
| Sodium Saccharin | 0.2000 |
| Menthol | 0.0200 |
| Titanium Dioxide | 1.0000 |
| | 100.0000 |

* A combination of 15% Sorbosil AC39 and 4% Zeodent 163

It should be understood that the above Example is illustrative of the best mode only of the invention herein disclosed. Given the present disclosure, it is anticipated that numerous variations will occur to those skilled in the art. A latitude of modification, substitution and change is intended and in some instances, some features of the invention will be employed without a corresponding use of other features. Accordingly, it is intended that the spirit and scope of the invention disclosed herein should be limited only by the following claims.

### Claims

1. An oral gel composition comprising in an orally acceptable vehicle the balanced admixture of at least two hydrated silicas of particular particle size, an anionic surfactant and a fluoridating agent.

2. An oral composition as claimed in claim 1, in which at least one of said hydrated silicas is a precipitated silica.

3. An oral gel composition as claimed in claim 1 or 2, which comprises a hydrated silica having an average particle size of from about 5 to about 15 microns, and a hydrated silica having an average particle size of from about 20 to about 40 microns.

4. An oral composition as claimed in claim 1, 2 or 3, in which said hydrated silica comprises from 20% to 50% by weight of the composition and 15% of said silica has a particle size of from 8 to 13 microns.

5. An oral composition as claimed in any one of the preceding claims, in which said anionic surfactant is sodium dodecylbenzenesulfonate.

6. An oral composition as claimed in any one of the preceding claims containing a fluoridating agent in an amount to provide a fluoride level of 1000 to 1500 ppm.

7. An oral composition as claimed in any one of the preceding claims further containing a desensitizing agent.

8.  An oral composition as claimed in claim 7, in which said desensitizing agent is selected from the group comprising potassium nitrate, dibasic sodium citrate, strontium chloride and formaldehyde.

9.  An oral gel composition as claimed in claim 8, in which said desensitizing agent is potassium nitrate.

**European Patent Office** EUROPEAN SEARCH REPORT

Application Number

EP 97 30 2486

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO 96 34592 A (PROCTER & GAMBLE) 7 November 1996<br>* claims 1,3,4,9 *<br>* page 10, line 13 - line 17 *<br>* page 11, line 16 - line 21 *<br>* page 14, line 19 - line 30 *<br>* page 15, line 16 - line 19 * | 1,2,6-9 | A61K7/16 |
| A | | 3,4 | |
| X | WO 96 34594 A (PROCTER & GAMBLE) 7 November 1996<br>* claims 1,2,4,8 *<br>* page 7, line 7 - line 12 *<br>* page 10, line 4 - line 15 * | 1,2,6 | |
| X | US 4 108 978 A (MAZZANOBILE SALVATORE ET AL) 22 August 1978<br>* column 3, line 10 - line 17 *<br>* column 3, line 33 - line 46 *<br>* column 3, line 61 - column 4, line 7; example 6 * | 1,3,6 | |
| X | WO 94 10969 A (HENKEL KGAA ;SCHUHMANN KLAUS (DE); FOERG FRANZ (DE); LASKA HANS (D) 26 May 1994<br>* page 2, line 23 - page 3, line 29; table 1 * | 1,6 | TECHNICAL FIELDS SEARCHED (Int.Cl.6)<br><br>A61K |
| A | CH 340 956 A (DURO-DONT GMBH) 31 October 1959<br>* example * | 1,3,4 | |

-/--

The present search report has been drawn up for all claims

| Place of search | Date of completion of the search | Examiner |
|---|---|---|
| BERLIN | 22 September 1997 | McConnell, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

**EUROPEAN SEARCH REPORT**

Application Number

EP 97 30 2486

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | DATABASE WPI<br>Section Ch, Week 8033<br>Derwent Publications Ltd., London, GB;<br>Class A96, AN 80-57547C<br>XP002039771<br>& JP 55 085 513 A (LION DENTIFRICE) , 27 June 1980<br>* abstract *<br>--- | 1,5 | |
| A | WO 93 25184 A (PROCTER & GAMBLE) 23 December 1993<br>* claims 1,5 *<br>* page 3, line 6 - page 4, line 3 *<br>--- | 1-3,6-9 | |
| A | WO 95 33441 A (BLOCK DRUG CO) 14 December 1995<br>* example 24 *<br>--- | 1,6-9 | |
| A | EP 0 543 442 A (UNILEVER NV ;UNILEVER PLC (GB)) 26 May 1993<br>* page 2, line 34 - line 55 *<br>* page 3, line 40 - line 48 *<br>--- | 1,2,6-8 | |
| A | DE 24 09 755 A (BLENDAX WERKE SCHNEIDER CO) 11 September 1975<br>--- | | |
| A | GB 1 264 292 A (UNILEVER LTD.) 16 February 1972 | | |
| D | & US 3 538 230 A<br>----- | | |

TECHNICAL FIELDS SEARCHED (Int.Cl.6)

The present search report has been drawn up for all claims

| Place of search | Date of completion of the search | Examiner |
|---|---|---|
| BERLIN | 22 September 1997 | McConnell, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)